Europäisches Patentamt

(19) European Patent Office

Office européen des brevets

(11) **EP 0 594 811 B1**

(12) **EUROPÄISCHE PATENTSCHRIFT**

(45) Veröffentlichungstag und Bekanntmachung des Hinweises auf die Patenterteilung:
**12.06.1996 Patentblatt 1996/24**

(21) Anmeldenummer: 93908916.5

(22) Anmeldetag: **06.04.1993**

(51) Int. Cl.$^6$: **A61K 7/13**

(86) Internationale Anmeldenummer:
**PCT/EP93/00848**

(87) Internationale Veröffentlichungsnummer:
**WO 93/23006 (25.11.1993 Gazette 1993/28)**

(54) **OXIDATIONSHAARFÄRBEMITTEL AUF DER BASIS EINER CREMEFÖRMIGEN TRÄGERMASSE UND VERFAHREN ZUM FÄRBEN VON HAAREN**

OXIDATION HAIR DYE BASED ON A CREAMY CARRIER AND PROCESS FOR DYEING HAIR

COLORANT D'OXYDATION POUR CHEVEUX, UN AGENT-SUPPORT SOUS FORME DE CREME, ET PROCEDE DE COLORATION DES CHEVEUX

(84) Benannte Vertragsstaaten:
**DE ES FR GB IT**

(30) Priorität: **18.05.1992 DE 4216381**

(43) Veröffentlichungstag der Anmeldung:
**04.05.1994 Patentblatt 1994/18**

(73) Patentinhaber: **Wella Aktiengesellschaft D-64295 Darmstadt (DE)**

(72) Erfinder:
• **MAGER, Herbert CH-1723 Marly (CH)**
• **PASQUIER, Gilbert CH-1724 Praroman (CH)**
• **HOCH, Dieter D-6102 Pfungstadt/Eich (DE)**
• **AEBY, Johann CH-1723 Marly (CH)**

(56) Entgegenhaltungen:
**EP-A- 0 166 100      WO-A-93/01792**
**DE-A- 3 834 142      DE-A- 4 017 718**
**GB-A- 2 018 302**

Bemerkungen:
Die Akte enthält technische Angaben, die nach dem Eingang der Anmeldung eingereicht wurden und die nicht in dieser Patentschrift enthalten sind.

**Beschreibung**

Oxidationshaarfärbemittel in Cremeform haben in der Haarfärbepraxis eine besondere Bedeutung erlangt. Solche Haarfärbemittel enthalten im Allgemeinen als Oxidationsfarbstoffe p-substituierte Benzolderivate wie zum Beispiel 2,5-Diaminotoluol, 4-Aminophenol, 1,4-Diaminobenzol, 2-(2'-Hydroxyethyl)-1,4-diaminobenzol und 4-Amino-N-(2'-mesylaminoethyl)anilin sowie 2,5-Diaminoanisol, 2,5-Diaminobenzylalkohol und Tetraaminopyrimidin. Diese werden als Entwicklersubstanzen bezeichnet. Die Entwicklersubstanzen werden in Kombination mit geeigneten Kupplersubstanzen eingesetzt. Als Kupplersubstanzen kommen insbesondere 1-Naphthol, Resorcin, 4-Chlorresorcin, m-Aminophenol, 5-Amino-o-kresol und Derivate des m-Phenylendiamins wie zum Beispiel 2-Amino-4-(2'-Hydroxyethylamino)anisol in Betracht. Durch geeignete Kombination von Entwickler- mit Kupplersubstanzen läßt sich eine breite Palette verschiedener Farbnuancen erzeugen.

Oxidationshaarfärbemittel bestehen aus zwei Komponenten, die kurz vor dem Gebrauch üblicherweise im Verhältnis 1:1 vermischt und dann auf das zu färbende Haar aufgetragen werden. Eine Komponente, die Farbträgermasse, enthält das Farbstoffgemisch und kann in Form einer Lösung, eines Gels oder bevorzugt als Creme vorliegen. Die andere Komponente liegt üblicherweise flüssig oder auch cremeförmig vor und enthält einen geeigneten oxidierenden Wirkstoff, zum Beispiel Wasserstoffperoxid.

Die Färbung entsteht im Haarschaft durch Reaktion der Entwickler- mit der Kupplersubstanz in Gegenwart des oxidierenden Wirkstoffs.

In der Haarfärbepraxis werden bei der Anwendung von Mischungen zweier cremeförmiger Komponenten und anschließender Auftragung mit dem Pinsel höherviskose Farbträgermassen auf Fettalkoholbasis bevorzugt. Diese haben jedoch den Nachteil, bei der Lagerung zusätzlich anzudicken, so daß die Entnahme aus der Tube erheblich erschwert wird und die Vermischung mit der den oxidierenden Wirkstoff enthaltenden Komponente mehr Zeit erfordert. Die üblichen cremeförmigen Farbträgermassen neigen außerdem bei der Entnahme aus der Tube zum "Fädenziehen", wodurch die Handhabung schwierig wird.

Die in der Praxis verwendeten hochviskosen Farbemulsionssysteme sind in produktionstechnischer Hinsicht sehr schwierig zu handhaben.

Dies zeigt sich im Herstellungsprozeß beispielsweise in einer erst bei relativ niedrigen Temperaturen eintretenden Emulsionsbildung, wodurch die Erstellung von Emulsionskonzentraten, die anschließend mit kaltem Wasser verdünnbar sind, nicht oder nur schwer möglich ist.

Aus der deutschen Offenlegungsschrift 3 834 142 ist ein lagerstabiles cremeförmiges Oxidationshaarfärbemittel mit hohem Farbstoff/Elektrolyt-Gehalt bekannt. Es handelt sich dabei um eine Zubereitung niedriger Viskosität mit einer hohen Anzahl an Inhaltsstoffen. Neben Fettalkoholen und Fettsäureestern enthält das Mittel ein Fettsäuremonoethanolamid, mit 2 Mol Ethylenoxid oxethylierten Laurylalkohol und bestimmte Emulgatoren. Mittel dieser Art werden bevorzugt unter Verwendung einer Schüttelflasche aufgetragen. Damit ist eine Anwendung durch den Färbekunden selbst nicht möglich. Bei Haarfärbemitteln niedriger Viskosität besteht, aufgrund verminderten Haftens am Haar, die Gefahr des Herabrinnens.

Aus der deutschen Offenlegungsschrift 40 17 718 ist ein Oxidationshaarfärbemittel bekannt, das durch Vermischen einer emulsionsförmigen Farbträgermasse und einer emulsionsförmigen oxidationsmittelhaltigen Zusammensetzung erhalten wird.

Dieses Mittel soll aufgrund der speziellen Verteilung der Fettalkoholmenge auf die Farbträgermasse und das Oxidationsmittel eine besonders leichte Vermischbarkeit der beiden Komponenten sowie eine erhöhte Viskosität aufweisen.

Aufgabe der Erfindung war es daher, ein neues cremeförmiges Oxidationshaarfärbemittel hoher Viskosität zur Verfügung zu stellen, das im Vergleich zu üblichen cremeförmigen Oxidationshaarfärbemitteln, lagerstabil und einfach zu handhaben ist. Zudem sollte die Herstellung der hochviskosen Farbträgermasse durch eine einfache Rezeptur erleichtert werden.

Es wurde nun gefunden, daß durch ein Mittel zur oxidativen Färbung von Haaren bestehend aus einer cremeförmigen Trägermasse und einem darin gelösten Farbstoffgemisch, dadurch gekennzeichnet, daß es

(A) 10 bis 30 Gewichtsprozent mindestens eines Fettalkohols mit 10 bis 24 Kohlenstoffatomen,

(B) 0,2 bis 6,0 Gewichtsprozent mindestens eines Diesters der Formel

$$R_1\text{-CO-O-}(CH_2\text{-}CH_2\text{-O})_n\text{-CO-}R_2 \qquad\qquad (I),$$

wobei n die Bedeutung 1, 2 oder 3 hat, sowie $R_1$ und $R_2$ gleiche oder verschiedene Alkylreste mit 12 bis 20 Kohlenstoffatomen darstellen,

(C) 0,5 bis 20 Gewichtsprozent Glycerinfettsäureester mit 10 bis 24 Kohlenstoffatomen,

(D) 0,1 bis 10 Gewichtsprozent nichtionische und/oder anionische und/oder ampholytische Emulgatoren,
enthält,
und

(E) einen pH-Wert von 4,5 bis 12,5 aufweist,

die gestellte Aufgabe in hervorragender Weise gelöst wird.

In dem neuen Oxidationshaarfärbemittel sind die Fettalkohole mit 10 bis 24 Kohlenstoffatomen, zum Beispiel Cetylstearylalkohol, als Verdicker enthalten. Die bevorzugte Menge des Fettalkohols beträgt 15 bis 25 Gewichtsprozent.

Als bevorzugter Diester der Formel (I) ist Ethylenglykoldistearat enthalten.

Die bevorzugte Menge an Glycerinfettsäureester beträgt 0,5 bis 10,0 Gewichtsprozent. Als Glycerinfettsäureester ist insbesondere Glycerinmono-distearat, vorzugsweise mit 30 bis 35 Gewichtsprozent Monoestergehalt, enthalten.

Der Gesamtgehalt an dem verdickend wirkenden Fettalkohol der Komponente (A) und den Fettsäureestern der Komponenten (B) und (C) beträgt vorzugsweise 25,0 bis 56,0 Gewichtsprozent.

Als Emulgatoren finden übliche nichtionische, anionische und ampholytische Emulgatoren, zum Beispiel Fettalkoholsulfate, hochethoxylierte Fettalkohole, Fettalkoholethansulfonsäuresalze, Cholesterin, zwitterionische Emulgatoren vom Betain-Typ und bevorzugt Fettalkoholethersulfate, vorzugsweise in Mengen von 2 bis 5 Gewichtsprozent, Anwendung. Die Hauptmenge des Emulgators ist vorzugsweise Natriumlaurylalkoholdiglykolethersulfat.

Ferner können zusätzlich als Verdicker Vaseline, Fettsäuren wie zum Beispiel Ölsäure und oxethylierte Nonylphenole in einer Menge von 1,0 bis 9,0 Gewichtsprozent enthalten sein.

Darüber hinaus kann das Oxidationshaarfärbemittel noch weitere für solche Mittel übliche Zusätze wie kationische Harze in einer Menge von 0,05 bis 1,0 Gewichtsprozent; Lösungsmittel, z. B. Ethanol, Isopropanol, Glycerin, 1,3-Butandiol und Propylenglykol, in einer Menge von 1,0 bis 5,0 Gewichtsprozent; Antioxidantien, in einer Menge von 0,01 bis 0,2 Gewichtsprozent; Parfümöle in einer Menge von 0,01 bis 1,0 Gewichtsprozent und Komplexbildner für Schwermetalle in einer Menge von 0,01 bis 0,5 Gewichtsprozent enthalten.

Das Oxidationshaarfärbemittel ist vorzugsweise frei von Alkylolamiden und niedrig oxethylierten Fettalkoholen wie z. B. mit 2 Mol Ethylenoxid oxethylierten Laurylalkohol.

Je nach Zusammensetzung kann das erfindungsgemäße Haarfärbemittel schwach sauer, neutral oder alkalisch eingestellt sein. Insbesondere weist es einen pH-Wert zwischen 4,5 und 12,5 auf, wobei die Einstellung vorzugsweise mit Ammoniak erfolgt.

Das in dem Oxidationshaarfärbemittel enthaltene Farbstoffgemisch besteht aus mindestens einer Kupplersubstanz und mindestens einer Entwicklersubstanz sowie gegebenenfalls zusätzlich mit sich selbst kuppelnden Farbstoffvorstufen und direkt auf das Haar aufziehenden Farbstoffen.

Die Entwicklersubstanzen und Kupplersubstanzen werden in dem Haarfärbemittel entweder als solche oder in Form ihrer physiologisch unbedenklichen Salze mit anorganischen oder organischen Säuren, wie zum Beispiel als Chlorid, Sulfat, Phosphat, Acetat, Propionat, Lactat oder Citrat, eingesetzt.

Die Kupplersubstanzen werden im allgemeinen in etwa äquimolarer Menge, bezogen auf die verwendeten Entwicklersubstanzen, eingesetzt. Wenn sich auch der äquimolare Einsatz als zweckmäßig erweist, so ist es doch nicht nachteilig, wenn die Kupplersubstanzen in einem gewissen Überschuß oder Unterschuß zum Einsatz kommen. Es ist ferner nicht notwendig, daß die Entwicklerkomponente und die Kupplerkomponente jeweils nur aus einem Farbstoff bestehen, vielmehr kann sowohl die Entwicklerkomponente ein Gemisch von bekannten Entwicklersubstanzen als auch die Kupplerkomponente ein Gemisch von bekannten Kupplersubstanzen darstellen.

Das Haarfärbemittel enthält als bekannte Kupplersubstanzen insbesondere 1-Naphthol, 4-Methoxy-1-naphthol, Resorcin, 4-Chlorresorcin, 4,6-Dichlorresorcin, 2-Methylresorcin, 3-Aminophenol, 3-Amino-6-methylphenol, 4-Hydroxy-1,2-methylendioxybenzol, 4-Amino-1,2-methylendioxybenzol, 4-(2'-Hydroxyethylamino)-1,2-methylendioxybenzol, 4-Hydroxyindol, 2,3-Diamino-6-methoxy-pyridin und 5-Amino-2-methylphenol. Weitere geeignete Kupplersubstanzen sind zum Beispiel 2,4-Dihydroxyphenolether wie 2,4-Dihydroxyanisol und 2,4-Dihydroxyphenoxyethanol.

Von den bekannten Entwicklersubstanzen kommen als Bestandteil des erfindungsgemäßen Haarfärbemittels vor allem 1,4-Diaminobenzol, 2,5-Diaminotoluol, 2,5-Diaminobenzylalkohol, 3-Methyl-4-aminophenol, 2-(2'-Hydroxyethyl)-1,4-diaminobenzol, Tetraaminopyrimidin und 4-Aminophenol in Betracht. Zur Haarfärbung bekannte und übliche Oxidationsfarbstoffe, die in dem Haarfärbemittel enthalten sein können, sind unter anderem in dem Buch von E. Sagarin, "Cosmetics, Science and Technology", Interscience Publishers Inc., New York (1957), Seiten 503 ff. sowie in dem Buch von H. Janistyn, "Handbuch der Kosmetika und Riechstoffe" (1973), Seiten 338 ff. beschrieben.

Die Gesamtmenge der in dem hier beschriebenen Haarfärbemittel enthaltenen Entwicklersubstanz-Kupplersubstanz-Kombination soll etwa 0,01 bis 12,0 Gewichtsprozent, insbesondere 0,2 bis 4,0 Gewichtsprozent, betragen.

Zur Erzielung gewisser Farbnuancen können ferner auch übliche direktziehende Farbstoffe, beispielsweise Triphenylmethanfarbstoffe wie Diamond Fuchsine (C.I. 42 510) und Leather Ruby HF (C.I. 42 520), aromatische Nitrofarbstoffe wie 2-Amino-4,6-dinitro-phenol, 2-Nitro-4-(2'-hydroxyethylamino)anilin, 2-N-2',3'-Dihydroxypropylamino-5-(N-methyl, N-hydroxyethyl)aminonitrobenzol und 2-Amino-4-nitrophenol, Azofarbstoffe wie Acid Brown 4 (C.I. 14 805) und Acid Blue 135 (C.I. 13 385), Anthrachinonfarbstoffe wie Disperse Violet 4 (C.I. 61 105), Disperse Blue 1 (C.I. 64 500), Disperse Red 15 (C.I. 60 710), Disperse Violet 1 (C.I. 61 100), außerdem 1,4,5,8-Tetraaminoanthrachinon und 1,4- Diaminoanthrachinon, enthalten sein.

Das Haarfärbemittel kann weiterhin auch mit sich selbst kuppelnde Farbstoffvorstufen, wie zum Beispiel 2-Amino-5-methylphenol, 2-Amino-6-methylphenol, 2-Amino-5-ethoxyphenol oder auch 2-Propylamino-5-aminopyridin, enthalten.

Die Gesamtmenge der direktziehenden Farbstoffe und der mit sich selbst kuppelnden Farbstoffvorstufen beträgt 0,01 bis 6 Gewichtsprozent, vorzugsweise 0,2 bis 4 Gewichtsprozent.

Die Gesamtmenge aller Farbstoffe, also der Entwicklersubstanz-Kupplersubstanz-Kombination, der mit sich selbst kuppelnden Farbvorstufen und der direktziehenden Farbstoffe, soll 0,1 bis 14 Gewichtsprozent, vorzugsweise 0,2 bis 8,0 Gewichtsprozent betragen.

Das erfindungsgemäße Oxidationshaarfärbemittel stellt ein Gemisch aus der Trägermasse und dem Farbstoffgemisch dar.

Bei der Anwendung vermischt man das Haarfärbemittel unmittelbar vor dem Gebrauch mit einem flüssigen oder cremeförmigen, einen oxidierenden Wirkstoff enthaltenden Oxidationsmittel in einem Gewichtsverhältnis von 2:1 bis 1:3. Im Oxidationsmittel können als weitere übliche Zusatzstoffe u.a. Emulgatoren und Netzmittel, Verdicker wie Fettalkohole, Säuren wie Phosphorsäure, Puffersubstanzen, Stabilisatoren wie Phenacetin, kationische Harze, Parfümöle, Acrylate und Trübungsmittel enthalten sein. Als oxidierender Wirkstoff zur Entwicklung der Haarfarbe kommt hauptsächlich Wasserstoffperoxid, beispielsweise als 6-%ige wäßrige Lösung bzw. dessen Additionsverbindungen an Harnstoff, Melamin oder Natriumborat, in Betracht. Das Wasserstoffperoxid ist in dem Oxidationsmittel, in einer Konzentration von 0,1 bis 20 Gewichtsprozent, bevorzugt 1,0 bis 12 Gewichtsprozent, enthalten.

Das gebrauchsfertige Gemisch aus dem Haarfärbemittel und dem Oxidationsmittel wird in einer für die Haarfärbung ausreichenden Menge, je nach Haarfülle etwa 90 bis 160 g, auf das Haar aufgetragen und bei 15 bis 50°C etwa 10 bis 45 Minuten lang, vorzugsweise 30 Minuten lang, einwirken gelassen. Man spült sodann das Haar mit Wasser aus und trocknet es. Gegebenenfalls wird im Anschluß an die Spülung mit Wasser noch mit einer schwachen physiologisch verträglichen organischen Säure, wie zum Beispiel Zitronensäure oder Weinsäure, nachgespült.

Bei dem erfindungsgemäßen Haarfärbemittel ist ein starkes Andicken wie bei herkömmlichen cremeförmigen Haarfärbemitteln, auch nach längerer Lagerung, nicht festzustellen. Das Mittel hat weiterhin die Eigenschaft, leicht eine stabile Emulsion zu bilden. Die Emulsionsbildung setzt schon bei relativ hohen Temperaturen ein, so daß die Erstellung eines Emulsionskonzentrats, das anschließend mit kaltem Wasser verdünnbar ist, möglich ist. Dies erleichtert die Herstellung cremeförmiger Haarfärbemittel erheblich.

Die nachfolgenden Beispiele für erfindungsgemäße Haarfärbemittel sollen den Gegenstand näher erläutern.

**Beispiel 1**

| 1. | Cetylstearylalkohol | 16,20 g |
|---|---|---|
| 2. | Natriumlaurylsulfat | 1,80 g |
| 3. | Glycerinmono-distearat | 7,50 g |
| 4. | Glycerin | 0,80 g |
| 4a. | Kaliumstearat | 0,55 g |
| 5. | Glykoldistearat | 1,80 g |
| 6. | Natriumlaurylalkoholdiglykolethersulfat, 26%-ige wäßrige Lösung | 10,00 g |
| 7. | Natriumsulfit, wasserfrei | 0,50 g |
| 8. | 2,5-Diaminotoluolsulfat | 1,20 g |
| 9. | Resorcin | 0,50 g |
| 10. | m-Aminophenol | 0,10 g |
| 11. | Ammoniak, 25%-ige wäßrige Lösung | 0,90 g |
| 12. | Wasser | 52,15 g |
| 13. | Ammoniak, 25%-ige wäßrige Lösung | 6,00 g |
| | | 100,00 g |

Die die Wachsphase bildenden Komponenten 1. bis 5. werden zusammen bei 90 °C geschmolzen. In die Schmelze wird die kochende wäßrige Lösung, bestehend aus den Rohstoffen 6. bis 12., eingerührt. Die so erhaltene Mischung wird unter Rühren erkalten gelassen, wobei sich ohne Schwierigkeiten eine Emulsion bildet. Nach dem Abkühlen der Creme wird der pH-Wert mit wäßriger Ammoniaklösung (13.) auf 10,3 eingestellt.

**Beispiel 2**

| 1. | Cetylstearylalkohol | 13,50 g |
|---|---|---|
| 2. | Natriumlaurylsulfat | 1,50 g |
| 3. | Glycerinmono-distearat | 11,30 g |
| 4. | Glycerin | 3,70 g |
| 4a. | Kaliumstearat | 0,82 g |
| 5. | Glykoldistearat | 1,80 g |
| 6. | Wasser | 30,00 g |
| 7. | Natriumlaurylalkoholdiglykolethersulfat, 26%-ige wäßrige Lösung | 10,00 g |
| 8. | Natriumsulfit, wasserfrei | 0,50 g |
| 9. | 2,5-Diaminotoluolsulfat | 1,60 g |
| 10. | Resorcin | 0,60 g |
| 11. | 3-Aminophenol | 0,20 g |
| 12. | Ammoniak, 25%-ige wäßrige Lösung | 1,20 g |
| 13. | Wasser | 17,28 g |
| 14. | Ammoniak, 25%-ige wäßrige Lösung | 6,00 g |
| | | 100,00 g |

In eine bei 90 °C erzeugten Schmelze der Komponenten 1. bis 5. wird unter Rühren eine 95 °C heiße wäßrige Lösung der Komponenten 6. bis 12. gegossen. Danach wird bei 40 - 60 °C Wasser (13.) in das Gemisch einfließen gelassen, womit eine schnellere Abkühlung verbunden ist. Die Emulsion bildet sich glatt. Die Einstellung des pH-Wertes auf 10,0 erfolgt mit wäßriger Ammoniaklösung (14.).

**Beispiel 3**

| | | |
|---|---|---|
| 1. | Cetylstearylalkohol | 18,45 g |
| 2. | Natriumlaurylsulfat | 2,05 g |
| 3. | Glycerinmono-distearat | 4,90 g |
| 4. | Glycerin | 1,60 g |
| 4a. | Kaliumstearat | 0,36 g |
| 5. | Cholesterin | 0,60 g |
| 6. | Glykoldistearat | 1,80 g |
| 7. | Cetylalkoholpolyethylenglykolether | 2,00 g |
| 8. | Natriumlaurylalkoholdiglykolethersulfat, 26%-ige wäßrige Lösung | 10,00 g |
| 9. | Ascorbinsäure | 0,30 g |
| 10. | Resorcin | 0,30 g |
| 11. | 2,5-Diaminotoluolsulfat | 0,60 g |
| 12. | Ammoniak, 25%-ige wäßrige Lösung | 6,00 g |
| 13. | Wasser | 51,04 g |
| | | 100,00 g |

Nachdem die Komponenten 1. bis 6. zusammen bei 90 °C geschmolzen wurden, wird die Emulsion wie in Beispiel 1 beschrieben hergestellt. 50 g der Haarfärbecreme werden mit 50 g 6%-iger Wasserstoffperoxidemulsion, folgender Zusammensetzung, kurz vor dem Gebrauch vermischt:

| | |
|---|---|
| Cetylstearylalkohol | 2,00 g |
| Natriumlaurylsulfat | 0,28 g |
| Cetylalkoholpolyethylenglykolether | 0,10 g |
| Wasserstoffperoxid, 50%-ig | 12,00 g |
| Phosphorsäure, 85%-ig | 0,10 g |
| Wasser | 85,62 g |
| | 100,00 g |

Das Gemisch wird anschließend auf ergrautes Haar aufgetragen und 30 Minuten lang bei Raumtemperatur einwirken gelassen. Danach wird das Haar mit Wasser gespült und getrocknet. Das Haar ist hellblond gefärbt.

**Vergleichsversuche**

Haarfärbemittel gemäß den Beispielen 1 bis 3 wurden mit Haarfärbemitteln gleicher Zusammensetzung, bei denen jedoch das Glykoldistearat mengengleich durch Wasser ersetzt wurde (Beispiele 1A, 2A und 3A), verglichen.

Im Fall von Beispiel 1A bildet sich, im Gegensatz zur erfindungsgemäßen Zusammensetzung nach Beispiel 1, schlecht eine Emulsion aus. Sie ist instabil und neigt zur Klumpenbildung und zu einer zeitweisen Phasentrennung. Während die erfindungsgemäße Farbträgermasse nach Beispiel 2 ohne Probleme eine stabile Emulsion bildet, tritt bei Beispiel 2A, welches keinen Glykoldiester enthält eine Phasentrennung auf.

Sowohl die Haarfärbecreme nach Beispiel 3, als auch die entsprechende nicht erfindungsgemäße Haarfärbecreme (Beispiel 3A), werden nach der Herstellung in Tuben abgefüllt und etwa 10 Tage gelagert. Bei der Entnahme aus der Tube ist im Falle der Farbmasse ohne einen Gehalt an Glykoldistearat (Beispiel 3A) ein Nachdicken und deutlich stärkeres "Fädenziehen" zu beobachten als bei der erfindungsgemäßen Haarfärbecreme (Beispiel 3).

Alle Prozentangaben stellen, soweit nicht anders vermerkt, Gewichtsprozente dar.

**Patentansprüche**

1. Mittel zum oxidativen Färben von Haaren bestehend aus einer cremeförmigen Trägermasse und einem darin gelösten Farbstoffgemisch, dadurch gekennzeichnet, daß es

   (A) 10 bis 30 Gewichtsprozent mindestens eines Fettalkohols mit 10 bis 24 Kohlenstoffatomen,

   (B) 0,2 bis 6,0 Gewichtsprozent mindestens eines Diesters der Formel

   $$R_1\text{-CO-O-}(CH_2\text{-}CH_2\text{-O})_n\text{-CO-}R_2 \qquad\qquad (I),$$

   wobei n die Bedeutung 1, 2 oder 3 hat, sowie $R_1$ und $R_2$ gleiche oder verschiedene Alkylreste mit 12 bis 20 Kohlenstoffatomen darstellen,

   (C) 0,5 bis 20 Gewichtsprozent Glycerinfettsäureester mit 10 bis 24 Kohlenstoffatomen,

   (D) 0,1 bis 10 Gewichtsprozent nichtionische und/oder anionische und/oder ampholytische Emulgatoren,
       enthält,
       und

   (E) einen pH-Wert von 4,5 bis 12,5 aufweist.

2. Mittel gemäß Anspruch 1, dadurch gekennzeichnet, daß es als Diester der Formel (I) Ethylenglykoldistearat enthält.

3. Mittel gemäß Anspruch 1 oder 2, dadurch gekennzeichnet, daß es als Glycerinfettsäureester Glycerinmonodistearat enthält.

4. Mittel gemäß Anspruch 3, dadurch gekennzeichnet, daß es das Glycerinmono-distearat in einer Menge von 0,5 bis 10 Gewichtsprozent enthält.

5. Mittel gemäß einem der Ansprüche 1 bis 4, dadurch gekennzeichnet, daß die Hauptmenge des Emulgators der Komponente (D) Natriumlaurylalkoholdiglykolethersulfat ist.

6. Mittel gemäß einem der Ansprüche 1 bis 5, dadurch gekennzeichnet, daß das Farbstoffgemisch mindestens eine der Kupplersubstanzen 1-Naphthol, 4-Methoxy-1-naphthol, Resorcin, 4-Chlorresorcin, 4,6-Dichlorresorcin, 2-Methylresorcin, 3-Aminophenol, 3-Amino-6-Methylphenol, 4-Hydroxy-1,2-methylendioxybenzol, 4-Hydroxyindol, 2,4-Dihydroxyanisol und 2,4-Dihydroxyphenoxyethanol enthält.

7. Mittel gemäß einem der Ansprüche 1 bis 6, dadurch gekennzeichnet, daß das Farbstoffgemisch mindestens eine der Entwicklersubstanzen 2,5-Diaminotoluol, 3-Methyl-4-aminophenol, 1,4-Diaminobenzol, 2-(2'-Hydroxyethyl)-1,4-diaminobenzol, Tetraaminopyrimidin und 4-Aminophenol enthält.

8. Mittel gemäß einem der Ansprüche 1 bis 7, dadurch gekennzeichnet, daß das Farbstoffgemisch mindestens einen der direktziehenden Farbstoffe Diamond Fuchsine (C.I. 42 510), Leather Ruby HF (C.I. 42 520), 2-Amino-4,6-dinitrophenol, 2-Nitro-4-(2'-hydroxyethylamino)anilin, 2-N-2',3'-Dihydroxypropylamino-5-(N-methyl,N-hydroxyethly)amino-nitrobenzol, 2-Amino-4-nitrophenol, Acid Brown 4 (C.I. 14 805), Acid Blue 135 (C.I. 13 385), Disperse Violet 4 (C.I. 61 105), Disperse Blue 1 (C.I. 64 500), Disperse Red 15 (C.I. 60 710), Disperse Violet 1 (C.I. 61 100), 1,4,5,8-Tetraaminoanthrachinon und 1,4-Diaminoanthrachinon enthält.

9. Verfahren zum oxidativen Färben von Haaren, dadurch gekennzeichnet, daß man ein Haarfärbemittel gemäß den Ansprüchen 1 bis 8 unmittelbar vor dem Gebrauch mit einem einen oxidierenden Wirkstoff, insbesondere Wasserstoffperoxid, enthaltenden flüssigen oder cremeförmigen Oxidationsmittel vermischt, anschließend eine für die Haarbehandlung ausreichende Menge dieses Gemisches auf das Haar aufträgt und bei 15 bis 50 Grad Celsius 10 bis 45 Minuten lang auf das Haar einwirken läßt, das Haar mit Wasser spült und sodann trocknet.

**Claims**

1. Agent for the oxidative dyeing of hair consisting of a carrier mass in cream form and a dye mixture dissolved therein, characterised in that it contains:

(A) 10 to 30 weight % of at least one fatty alcohol with 10 to 24 carbon atoms;

(B) 0.2 to 6.0 weight % of at least one diester of the formula

$$R_1\text{-CO-O-}(CH_2\text{-}CH_2\text{-O})_n\text{-CO-}R_2 \qquad\qquad (I),$$

in which n signifies 1, 2 or 3 and $R_1$ and $R_2$ are identical or different alkyl residues with 12 to 20 carbon atoms;

(C) 0.5 to 20 weight % of glycerol fatty acid ester with 10 to 24 carbon atoms;

(D) 0.1 to 10 weight % of non-ionic and/or anionic and/or ampholytic emulsifiers; and

(E) has a pH value of from 4.5 to 12.5.

2. Agent according to Claim 1, characterised in that it contains ethylene glycol distearate as the diester of formula (I).

3. Agent according to Claim 1 or 2, characterised in that it contains glycerol mono distearate as the glycerol fatty acid ester.

4. Agent according to Claim 3, characterised in that it contains the glycerol mono distearate in an amount of from 0.5 to 10 weight %.

5. Agent according to any one of Claims 1 to 4, characterised in that the principal amount of the emulsifier of component (D) is sodiumlaurylalcoholdiglycolether sulphate.

6. Agent according to any one of Claims 1 to 5, characterised in that the dye mixture contains at least one of the coupler substances 1-naphthol, 4-methoxy-1-naphthol, resorcinol, 4-chlororesorcinol, 4,6-dichlororesorcinol, 2-methylresorcinol, 3-aminophenol, 3-amino-6-methylphenol, 4-hydroxy-1,2-methylenedioxybenzene, 4-hydroxyindole, 2,4-dihydroxyanisole and 2,4-dihydroxyphenoxyethanol.

7. Agent according to any one of Claims 1 to 6, characterised in that the dye mixture contains at least one of the developer substances 2,5-diaminotoluene, 3-methyl-4-aminophenol, 1,4-diaminobenzene, 2-(2'-hydroxyethyl)-1,4-diaminobenzene, tetraaminopyrimidine and 4-aminophenol.

8. Agent according to any one of Claims 1 to 7, characterised in that the dye mixture contains at least one of the direct-acting dyes Diamond Fuchsine (C.I. 42 510), Leather Ruby HF (C.I. 42 520), 2-amino-4,6-dinitrophenol, 2-nitro-4-(2'-hydroxyethylamino)aniline, 2-N-2',3'-dihydroxypropylamino-5-(N-methyl,N-hydroxyethyl)aminonitro-benzene, 2-amino-4-nitrophenol, Acid Brown 4 (C.I. 14 805), Acid Blue 135 (C.I. 13 385), Disperse Violet 4 (C.I. 61 105), Disperse Blue 1 (C.I. 64 500), Disperse Red 15 (C.I. 60 710), Disperse Violet 1 (C.I. 61 100), 1,4,5,8-tetraamino-anthraquinone and 1,4-diamino-anthraquinone.

9. Process for the oxidative dyeing of hair, characterised in that a hair colouring agent according to any one of Claims 1 to 8 is mixed immediately before use with a liquid or cream oxidising agent containing an oxidising substance, in particular hydrogen peroxide, subsequently an amount of this mixture sufficient for treating the hair is applied to it and is left to act on the hair at from 15 to 50°C for from 10 to 45 minutes, the hair is rinsed with water and then dried.

**Revendications**

1. Agent de teinture capillaire oxydant se composant d'une matière porteuse et d'un mélange colorant qui y est dissous, caractérisé en ce qu'il contient:

(A) 10 à 30% en poids d'un alcool gras avec 10 à 24 atomes de carbone;

(B) 0,2 à 6,0% en poids d'un diester de formule

$$R1\text{-CO-O-}(CH2\text{-}CH2\text{-O})n\text{-CO-}R2 \qquad\qquad (I)$$

dans laquelle n représente 1, 2 ou 3, R1 et R2, identiques ou différents, représentent un reste alkyle avec 12 à 20 atomes de carbone;

(C) 0,5 à 20% en poids d'un ester d'acide gras de la glycérine, contenant 10 à 24 atomes de carbone;

(D) 0,1 à 10% en poids d'un émulsifiant non ionique et/ou anionique et/ou ampholyte; et

(E) présente une valeur de pH allant de 4,5 à 12,5.

2. Agent selon la revendication 1, caractérisé en ce qu'il contient comme diester de formule (I) un distéarate d'éthylène-glycol.

3. Agent selon la revendication 1 ou 2, caractérisé en ce qu'il contient comme ester d'acide gras de la glycérine un monodistéarate de glycérine.

4. Agent selon la revendication 3, caractérisé en ce qu'il contient le monodistéarate de glycérine à raison de 0,5 à 10% en poids.

5. Agent selon l'une des revendications 1 à 4, caractérisé en ce que la quantité principale de l'émulsifiant des composants (D) est un éther-sulfate de lauryloxydiglycol et de sodium.

6. Agent selon l'une des revendications 1 à 5, caractérisé en ce que le mélange colorant contient au moins un des copulants suivants: le 1-naphtol, le 4-méthoxy-1-naphtol, la résorcine, la 4-chlororésorcine, la 4,6-dichloro-résorcine, la 2-méthyl-résorcine, le 3-aminophénol, le 3-amino-6-méthyl-phénol, le 4-hydroxy-1,2-méthylène-dioxybenzène, le 4-hydroxyindol, le 2,4-dihydroxyanisol et le 2,4 dihydroxyphénoxyétanol.

7. Agent selon l'une des revendications 1 à 6, caractérisé en ce que le mélange colorant contient au moins une des substances de développement suivantes: le 2,5-diaminotoluène, le 3-méthyl-4-aminophénol, le 1,4-diaminobenzène, le 2-(2'-hydroxyéthyl)-1,4-diaminobenzène, la tétraaminopyrimidine et le 4-aminophénol.

8. Agent selon l'une des revendications 1 à 7, caractérisé en ce que le mélange colorant contient au moins un des colorants directs suivants: la "Diamond Fuchsine" (C.I. 42 510), le "Leather Ruby HF" (C.I. 42 520), le 2-amino-4,6-dinitrophénol, la 2-nitro-4-(2'-hydroxyéthylamino)aniline, le 2-N-2',3'-dihydroxypropylamino-5-(N-méthyl,N-hydroxyéthylaminonitro-benzène, le 2-amino-4-nitrophénol, l'Acide Brown 4 (C.I. 14 805), l'Acide Blue 135 (C.I. 13 385), le Dispers Violet 4 (C.I. 61 105), le Dispers Blue 1 (C.I. 64 500), le Dispers Red 15 (C.I. 60 710), le Dispers Violet 1 (C.I. 61 100), la 1,4,5,8-tétraaminoanthraquinone, et la 1,2-diamino-anthraquinone.

9. Procédé de coloration oxydante des cheveux, caractérisé en ce que l'on mélange un agent de teinture capillaire selon les revendications 1 à 8, immédiatement avant utilisation, avec un agent oxydant, en particulier du peroxyde d'hydrogène, contenant un agent d'oxydation liquide ou sous forme de crème, puis pour le traitement des cheveux, on applique sur ces derniers, une quantité suffisante de ce mélange et on laisse agir entre 15 et 50°C pendant 10 à 45 minutes, on rince les cheveux à l'eau puis on sèche.